# EUROPÄISCHE PATENTANMELDUNG

(11) **EP 0 863 124 A1**
(43) Veröffentlichungstag der Anmeldung: **09.09.1998**
(21) Anmeldenummer: 98103923.3
(22) Anmeldetag: 05.03.1998
(51) Int. Cl.: C07C 51/48, C07C 57/07

(54) **Verfahren zur Herstellung von (Meth)acrylsäure**

(30) Priorität: 07.03.1997 DE 19709471
(71) Anmelder: BASF AKTIENGESELLSCHAFT, 67056 Ludwigshafen (DE)
(72) Erfinder: Schraut, Armin, Dr., 64625 Bensheim (DE); Ulbrich, Michael-Dieter, Dr., 67251 Freinsheim (DE); Martin, Friedrich-Georg, Dr., 69124 Heidelberg (DE); Eck, Bernd, 68519 Viernheim (DE); Heilek, Jörg, 69245 Bammental (DE)
(74) Vertreter: Isenbruck, Günter, Dr.

(57) **Zusammenfassung**

Verfahren zur Herstellung von (Meth)acrylsäure, das die folgenden Stufen umfaßt:
A: Herstellung eines Reaktionsgases, das (Meth)acrylsäure, ein Alkan, Alkanol, Alken, oder Alkenal mit 3 oder 4 C-Atomen oder ein Gemisch aus zwei oder mehr davon, Wasser und Sauerstoff enthält, durch heterogen-katalysierte Gasphasenoxidation eines Alkans, Alkanols, Alkens oder Alkenals mit 3 oder 4 C-Atomen oder einem Gemisch aus zwei oder mehr davon;
B: Kondensation des in Stufe A erhaltenen Reaktionsgases, wobei eine erste wäßrige Lösung, die die Hauptmenge an (Meth)acrylsäure und eine Restmenge an Alkan, Alkanol, Alken, Alkenal mit 3 oder 4 C-Atomen oder des Gemischs aus zwei oder mehr davon aus dem Reaktionsgas enthält, und eine erste Gasphase, die die Hauptmenge an Alkan, Alkanol, Alken, Alkenal mit 3 oder 4 C-Atomen oder des Gemischs aus zwei oder mehr davon und eine Restmenge an (Meth)acrylsäure aus dem Reaktionsgas enthält, erhalten wird;
C: Absorption der in der ersten Gasphase enthaltenen (Meth)acrylsäure durch Inkontaktbringen der ersten Gasphase mit Frischwasser, wobei eine zweite wäßrige Lösung, die (Meth)acrylsäure enthält, und eine zweite Gasphase, die das Alkan, Alkanol, Alken oder Alkenal mit 3 oder 4 C-Atomen oder das Gemisch aus zwei oder mehr davon enthält, erhalten wird;
D: Extraktion der zweiten wäßrigen Lösung, die (Meth)acrylsäure enthält, durch Inkontaktbringen mit einer Lösung, die ein Alkan, Alkanol, Alken oder Alkenal mit 3 oder 4 C-Atomen oder ein Gemisch aus zwei oder mehr davon enthält, und mit der zweiten wäßrigen Lösung eine Mischungslücke bildet, wobei eine organische Phase, die (Meth)acrylsäure und den Hauptteil an Alkan, Alkanol, Alken, Alkenal mit 3 oder 4 C-Atomen oder des Gemischs aus zwei oder mehr davon enthält, und eine dritte wäßrige Lösung, die einen geringeren Teil an Alkan, Alkanol, Alken, Alkenal mit 3 oder 4 C-Atomen oder dem Gemisch aus zwei oder mehr davon enthält, erhalten wird.

## Beschreibung

Die vorliegende Erfindung betrifft ein neues Verfahren zur Herstellung von (Meth)acrylsäure, das eine Stufe umfaßt, in der eine wäßrige Lösung, die (Meth)acrylsäure enthält, durch Inkontaktbringen mit einer Lösung, die mindestens ein Edukt für die Herstellung von (Meth)acrylsäure enthält, und mit der wäßrigen Lösung eine Mischungslücke bildet, extrahiert wird.

Der im Rahmen dieser Anmeldung verwendete Begriff "(Meth)acrylsäure" bezeichnet sowohl Acrylsäure als auch Methacrylsäure.

Methacrylsäure bildet aufgrund ihrer sehr reaktionsfähigen monoethylenisch ungesättigten Bindung sowie der Säurefunktion ein wertvolles Monomer zur Herstellung von Polymerisaten, wie z.B. für als Klebstoffe geeignete wäßrige Polymerdispersionen.

Unter anderem ist Methacrylsäure zugänglich durch Gasphasenoxidation von 1-Buten, Isobuten, Isobutyraldehyd, Isobuttersäure, Isobuten, MTBE und/oder Methacrolein mit Sauerstoff oder Sauerstoff-enthaltenden Gasen in Gegenwart von Katalysatoren, wie z.B. Multimetalloxiden, die die Elemente Molybdän und Vanadium in oxidischer Form enthalten. Die Oxidation wird bei erhöhter Temperatur sowie infolge der hohen Reaktionswärme vorzugsweise unter Verdünnen der Reaktionspartner mit Inertgasen wie N₂, CO₂ und/oder Kohlenwasserstoffen und/oder Wasserdampf durchgeführt. Bei diesen Verfahren wird jedoch nicht reine Methacrylsäure, sondern ein Reaktionsgasgemisch, das neben Methacrylsäure die Ausgangsstoffe, beispielsweise nicht umgesetztes Methacrolein, Wasserdampf, inertes Verdünnungsgas (z.B. Stickstoff), und Nebenprodukte (z.B. Kohlenoxide), niedere Aldehyde, wie z.B. Formaldehyd, hochsiedende Bestandteile, wie z.B. Citraconsäure, und insbesondere Essigsäure enthält, erhalten, von welchem die Methacrylsäure anschließend abgetrennt werden muß (vgl. z.B. EP-A 253 409 und DE-A 19 62 431). Als Ausgangsverbindungen sind aber auch solche denkbar, aus welchen sich die eigentliche C₄-Ausgangsverbindung, z.B. Methacrolein, während der Gasphasenoxidation erst intermediär bildet. Beispielhaft genannt sei der Methylether des tert.-Butanols (MTBE).

Ferner ist es auch möglich zur Herstellung von Methacrolein Formaldehyd und Propionaldehyd zu kondensieren und durch Destillation Methacrolein in, verglichen mit den vorher besprochenen Verfahren, höherer Reinheit zu erhalten. Ein derartiges Verfahren wird in der EP-B 58 927 beschrieben. Das so gewonnene Methacrolein kann danach in herkömmlicher Weise durch katalytische Gasphasenoxidation zu Methacrylsäure umgesetzt werden. Eine derartige Umsetzung wird unter anderem in der EP-B 297 445 beschrieben.

Die Gewinnung von Acrylsäure ist ausgehend von den entsprechenden C₃-Verbindungen, insbesondere Propylen und/oder Acrolein, möglich.

Will man in einem Verfahren zur Herstellung von (Meth)acrylsäure diese durch Extraktion aus dem erhaltenen Reaktionsgasgemisch abtrennen, wird das Reaktionsgasgemisch zunächst einer Kondensationsstufe unterworfen und anschließend extrahiert. So beschreibt die EP-B 345 083 ein derartiges Verfahren, das eine Methacrylsäure-Extraktionsstufe umfaßt, in der Methacrylsäure mit einem gesättigten Kohlenwasserstoff mit 6 bis 9 Kohlenstoffatomen extrahiert wird.

Gemäß der EP-A 710 643 wird innerhalb eines Verfahrens zur Aufreinigung von Methacrylsäure eine durch Kühlen und Kondensieren des Reaktionsgases erhaltene wäßrige Methacrylsäure-Lösung durch Zugabe eines organischen Lösungsmittels, vorzugsweise einem aliphatischen Kohlenwasserstoff mit 5 bis 9 Kohlenstoffatomen, einem aromatischen Kohlenwasserstoff, einem Ester oder einem Gemisch davon, aus der wäßrigen Lösung extrahiert.

Das japanische Patent JP 57 095 938 beschreibt die Extraktion von Acrylsäure aus einer verdünnten wäßrigen Lösung durch Extraktion mittels einem Sauerstoff-enthaltenden Lösungsmittel und einem tertiären Amin. Beispielhaft sind Trioctylamin und 2,6-Dimethyl-4-heptanol genannt.

Alle diese Verfahren sind jedoch dahingehend nachteilig, daß zur Extraktion von (Meth)acrylsäure jeweils ein Fremdstoff eingesetzt wird, der zum einen nicht ohne zusätzliche Kosten zugänglich ist und darüber hinaus innerhalb eines derartigen Verfahrens zusätzliche Schritte erfordert, um diesen Fremdstoff wieder abzutrennen, was zusätzliche Kosten und zusätzlichen Energieaufwand mit sich bringt.

Keines der aus dem Stand der Technik bekannten Verfahren beschreibt die Möglichkeit einer Extraktion von (Meth)acrylsäure mittels einer Lösung, die ein Alkan, Alkanol, Alken, oder Alkenal mit 3 oder 4 C-Atomen oder ein Gemisch aus zwei oder mehr davon, also ein Edukt zur Herstellung von (Meth)acrylsäure enthält. Als Extraktionsmittel werden bislang lediglich Fremdstoffe, wie oben erwähnt, verwendet.

Der vorliegenden Erfindung liegt somit die Aufgabe zugrunde, ein Verfahren zur Herstellung von (Meth)acrylsäure bereitzustellen, mit dem die oben beschriebenen Nachteile der bisher beschriebenen Verfahren zur Herstellung von (Meth)acrylsäure ganz oder teilweise vermieden werden können. Ein besonderes Augenmerk der vorliegenden Erfindung liegt dabei auf einer Extraktion von (Meth)acrylsäure ohne die Verwendung von Fremdsubstanzen. Ferner sollte es im Rahmen dieses Verfahrens möglich sein, mit einer - verglichen mit den Verfahren gemäß des Standes der Technik - geringeren Menge an Wasser und Energie auszukommen. Auch diese beiden Aufgaben werden durch das erfindungsgemäße Verfahren erfüllt, das, falls dies erwünscht ist, ganz ohne Destillationsstufen, die bekanntermaßen sehr energieintensiv sind, durchgeführt werden kann.

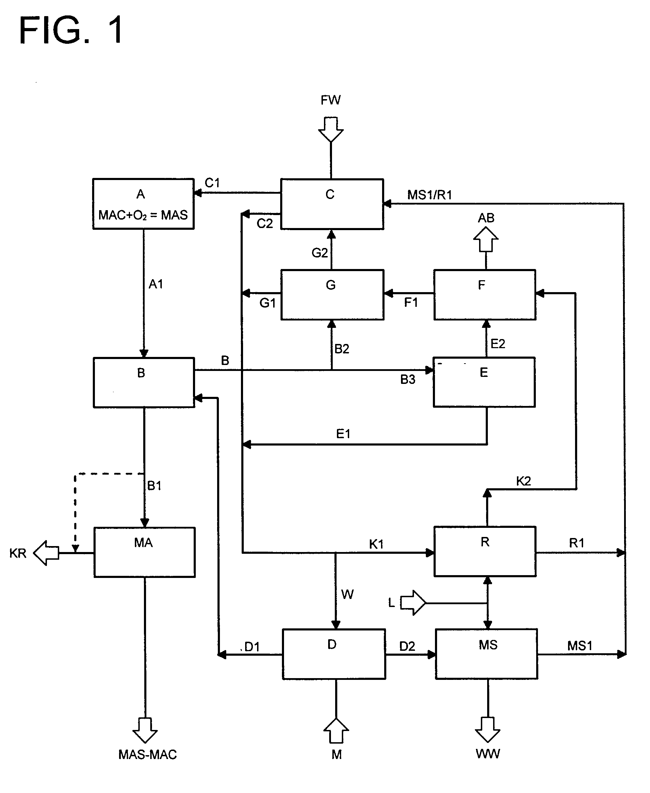

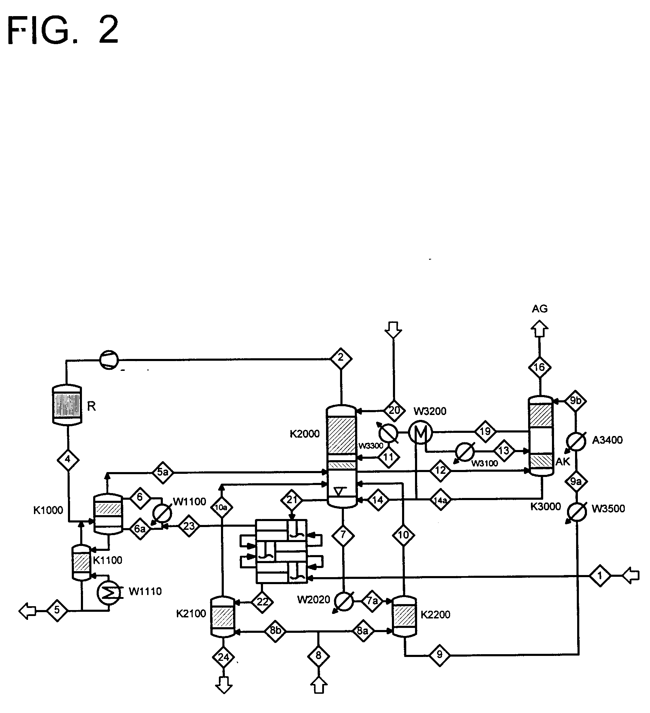

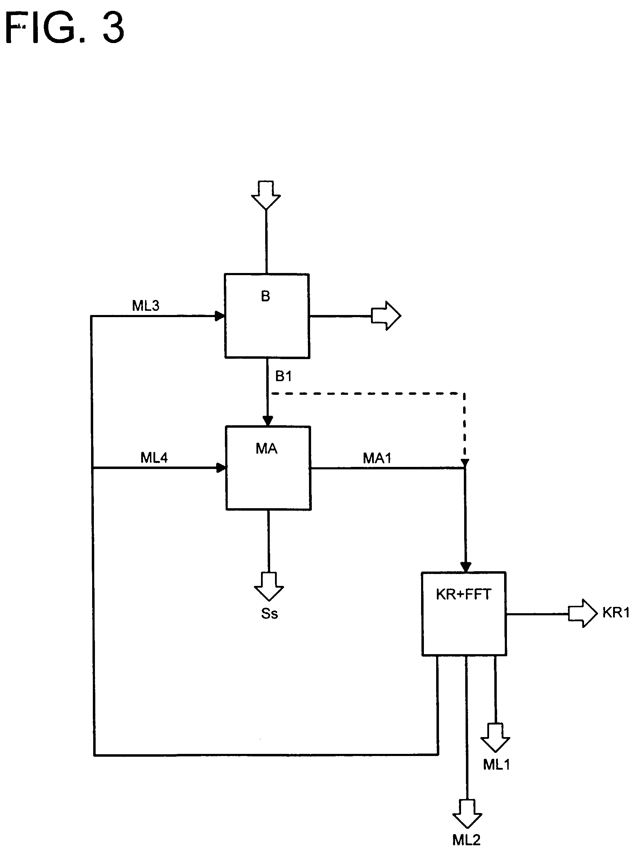

Zur besseren Illustration der vorliegenden Erfindung sind dieser Anmeldung drei Figuren beigefügt. Fig. 1 stellt eine schematische Darstellung einer bevorzugten Ausführungsform des erfindungsgemäßen Verfahrens dar und wird im Rahmen der Beschreibung näher erläutert. Fig. 2 stellt ein Verfahrensfließbild mit den wichtigsten Apparaten dar und wird bei der Diskussion des erfindungsgemäßen Beispiels naher erläutert. Fig. 3 stellt eine Ergänzung zu Fig. 1 dar und veranschaulicht die mögliche Einbindung einer Kristallisationsstufe zum Erhalt von Rein-(Meth)acrylsäure in das erfindungsgemäße Verfahren.

Die vorliegende Erfindung betrifft somit ein Verfahren zur Herstellung von (Meth)acrylsäure, das die folgenden Stufen umfaßt:
A: Herstellung eines Reaktionsgases, das (Meth)acrylsäure, ein Alkan, Alkanol, Alken, oder Alkenal mit 3 oder 4 C-Atomen oder ein Gemisch aus zwei oder mehr davon, Wasser und Sauerstoff enthält, durch heterogen-katalysierte Gasphasenoxidation eines Alkans, Alkanols, Alkens oder Alkenals mit 3 oder 4 C-Atomen oder einem Gemisch aus zwei oder mehr davon;
B: Kondensation des in Stufe A erhaltenen Reaktionsgases, wobei eine erste wäßrige Lösung, die die Hauptmenge an (Meth)acrylsäure und eine Restmenge an Alkan, Alkanol, Alken, Alkenal mit 3 oder 4 C-Atomen oder dem Gemisch aus zwei oder mehr davon aus dem Reaktionsgas enthält, und eine erste Gasphase, die die Hauptmenge an Alkan, Alkanol, Alken, Alkenal mit 3 oder 4 C-Atomen oder dem Gemisch aus zwei oder mehr davon und eine Restmenge an (Meth)acrylsäure aus dem Reaktionsgas enthält, erhalten wird;
C: Absorption der in der ersten Gasphase enthaltenen (Meth)acrylsäure durch Inkontaktbringen der ersten Gasphase mit Frischwasser, wobei eine zweite wäßrige Lösung, die (Meth)acrylsäure enthält, und eine zweite Gasphase, die das Alkan, Alkanol, Alken oder Alkenal mit 3 oder 4 C-Atomen oder das Gemisch aus zwei oder mehr davon enthält, erhalten wird;
D: Extraktion der zweiten wäßrigen Lösung, die (Meth)acrylsäure enthält, durch Inkontaktbringen mit einer Lösung, die ein Alkan, Alkanol, Alken, oder Alkenal mit 3 oder 4 C-Atomen oder ein Gemisch aus zwei oder mehr davon enthält, und mit der zweiten wäßrigen Lösung eine Mischungslücke bildet, wobei eine organische Phase, die (Meth)acrylsäure und den Hauptteil an Alkan, Alkanol, Alken, Alkenal mit 3 oder 4 C-Atomen oder des Gemischs aus zwei oder mehr davon enthält, und eine dritte wäßrige Lösung, die einen geringeren Teil an Alkan, Alkanol, Alken, Alkenal mit 3 oder 4 C-Atomen oder des Gemischs aus zwei oder mehr davon enthält, erhalten wird.

Wie sich aus obigem ergibt, besteht das erfindungsgemäße Verfahren aus den Stufen A bis D, wobei insbesondere die Extraktion von (Meth)acrylsäure mittels eines Alkans, Alkanols, Alkens oder Alkenats mit 3 oder 4 C-Atomen oder des Gemischs aus zwei oder mehr davon als Extraktionsmittel (Stufe D) als erfindungswesentlich anzusehen ist. Die restlichen, im folgenden beschriebenen Stufen E bis G und KR + FFT sowie die hierin beschriebenen Modifikationen bzw. Zwischenstufen können mit dem Verfahren mit den Stufen A bis D in für den Fachmann ersichtlicher Weise - je nach Anwendungsart und -zweck - in technisch sinnvoller Weise beliebig kombiniert werden. Die folgende Beschreibung sowie die Unteransprüche betreffen bevorzugte Ausführungsformen dieser möglichen Kombinationen.

Die Herstellung eines Reaktionsgases, das (Meth)acrylsäure, (Meth)acrolein, Wasser und Sauerstoff enthält, gemäß Stufe A, ist an sich bekannt und wird u.a. in der DE-A 44 05 059, EP-A 353 409, EP-A 92 097 und DE-A 44 31 949 beschrieben.

So kann (Meth)acrylsäure unter anderem durch katalytische Gasphasenoxidation eines Alkans, Alkanols, Alkens oder Alkenals mit 3 oder 4 C-Atomen oder eines Gemischs aus zwei oder mehr davon hergestellt werden. Besonders vorteilhaft läßt sich (Meth)acrylsäure z.B. durch katalytische Gasphasenoxidation von Propen, Acrolein, tert. Butanol, Iso-Buten, Iso-Butan, Iso-Butyraldehyd oder (Meth)acrolein herstellen. Dabei wird in einer bevorzugten Ausführungsform das (Meth)acrolein durch C1/C3-Kondensation, wie in der EP-B 58 927 beschrieben, hergestellt.

Als Ausgangsverbindungen sind aber auch solche denkbar, aus welchen sich die eigentliche C₃-/C₄-Ausgangsverbindung während der Gasphasenoxidation erst intermediär bildet. Beispielhaft genannt sei der Methylether des tert. Butanols.

Dabei werden diese Ausgangsstoffe, in der Regel mit inerten Gasen wie Stickstoff, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z.B. Mo, V, W und/oder Fe enthaltende) Mischoxidkatalysatoren geleitet und oxidativ in die (Meth)acrylsäure umgewandelt.

Aufgrund zahlreicher im Verlauf der katalytischen Gasphasenoxidation erfolgender Parallel- und Folgereaktionen sowie aufgrund der mitzuverwendenden inerten Verdünnungsgase, wird bei der katalytischen Gasphasenoxidation jedoch keine reine (Meth)acrylsäure, sondern ein Reaktionsgemisch erhalten, das im wesentlichen (Meth)acrylsäure, die inerten Verdünnungsgase, das Alkan, Alkanol, Alken oder Alkenal mit 3 oder 4 C-Atomen oder das Gemisch aus zwei oder mehr davon und Nebenprodukte, wie z.B. Wasser und Essigsäure enthält, aus welchem die (Meth)acrylsäure abgetrennt werden muß.

Dies geschieht im Rahmen des erfindungsgemäßen Verfahrens zunächst durch Kondensation des in Stufe A erhaltenen Reaktionsgases (Stufe B). Dabei wird eine erste wäßrige Lösung, die die Hauptmenge an (Meth)acrylsäure und eine Restmenge an Alkan, Alkanol, Alken, Alkenal mit 3 oder 4 C-Atomen oder des Gemischs aus zwei oder mehr davon aus dem Reaktionsgas enthält, und eine erste Gasphase, die die Hauptmenge an Alkan, Alkanol, Alken, Alkenal mit 3 oder 4 C-Atomen oder des Gemischs aus zwei oder mehr davon und eine Restmenge an (Meth)acrylsäure aus dem Reaktionsgas enthält, erhalten.

Derartige Kondensationen von bei der Gasphasenoxidation zur Herstellung von (Meth)acrylsäure erhaltenen Gemischen sind aus dem Stand der Technik ebenfalls bekannt und werden beispielsweise in der DE-OS 37 21 865 und der DE-OS 42 25 321 beschrieben.

Bezüglich der Temperatur, die bei der Kondensation im Rahmen des erfindungsgemäßen Verfahrens verwendet werden kann, existieren keine besonderen Beschränkungen, solange die Temperatur so eingestellt wird, daß eine erste wäßrige Lösung, die die Hauptmenge an (Meth)acrylsäure und eine Restmenge an Alkan, Alkanol, Alken, Alkenal mit 3 oder 4 C-Atomen oder des Gemischs aus zwei oder mehr davon enthält, erhalten wird. Im allgemeinen wird bei einem Absolutdruck von 1 bis 1,5 bar die Kondensation bei einer Temperatur von ungefähr 100°C oder weniger, vorzugsweise ungefähr 0 bis ungefähr 100°C, weiter bevorzugt bei ungefähr 30 bis ungefähr 80°C durchgeführt. Bei Wahl eines anderen Druckbereiches sind die Temperaturbereiche anzupassen.

Der Begriff "Hauptmenge" bedeutet im Rahmen der vorliegenden Anmeldung, daß das Konzentrationsverhältnis (Meth)acrylsäure/Alkan, Alkanol, Alken, Alkenal mit 3 oder 4 C-Atomen oder Gemisch aus zwei oder mehr davon größer ist als im gemäß Stufe A erhaltenen Reaktionsgas, d.h. die Menge an vorhandener (Meth)acrylsäure ist gegenüber der im Reaktionsgas vorhandenen Menge an (Meth)acrylsäure erhöht. Der Begriff "Restmenge" bedeutet, daß das Konzentrationsverhältnis (Meth)acrylsäure/Alkan, Alkanol, Alken, Alkenal mit 3 oder 4 C-Atomen oder Gemisch aus zwei oder mehr davon kleiner ist als im gemäß Stufe A erhaltenen Reaktionsgas, d.h. die Menge an (Meth)acrylsäure ist gegenüber der Menge derselben im Reaktionsgas erniedrigt.

In einer weiteren Ausführungsform der im Rahmen der vorliegenden Erfindung durchgeführten Kondensation wird das Reaktionsgas bei den obengenannten Temperaturen in direkten Gegenstrom-Kontakt mit einem Teil der vorher bei der Kondensation erhaltenen ersten wäßrigen Lösung oder aber einer von außen zugeführten wäßrigen Lösung, die (Meth)acrylsäure enthält, gebracht.

Die Kondensation kann ein- oder mehrstufig durchgeführt werden.

Zur Durchführung der oben beschriebenen Kondensation können beliebige herkömmliche Apparaturen, wie z.B. Füllkörper-, Siebboden-, Glockenboden- oder Sprühtürme, verwendet werden. Genauere Angaben bezüglich der verwendbaren Apparaturen lassen sich Ullmann's Enzyklopädie der Technischen Chemie 4. Aufl., Band 3, Seite 357 - 395 "Reaktionsapparate für Gas-Flüssig-Reaktion" entnehmen.

Ferner umfaßt das erfindungsgemäße Verfahren die Absorption der in der ersten Gasphase enthaltenen (Meth)acrylsäure durch Inkontaktbringen dieser Gasphase mit Frischwasser, wobei eine zweite wäßrige Lösung, die (Meth)acrylsäure enthält und eine zweite Gasphase, die Alkan, Alkanol, Alken, Alkenal mit 3 oder 4 C-Atomen oder ein Gemisch aus zwei oder mehr davon enthält, erhalten wird.

Um die Menge an im Rahmen des vorliegenden Verfahrens aufzuarbeitenden und schlußendlich verbrauchten Wassers möglichst gering zu halten, wird vorzugsweise die Menge an Frischwasser so eingestellt, daß die Konzentration der (Meth)acrylsäure in der zweiten wäßrigen Lösung ungefähr 1 bis ungefähr 80 und weiter bevorzugt ungefähr 5 bis ungefähr 25 Gew.-% beträgt, solange der Absolutdruck 1 bis 1,5 bar beträgt.

Die Absorption gemäß Stufe C wird im allgemeinen bei einer Temperatur von ungefähr 20 bis ungefähr 100°C, vorzugsweise ungefähr 50 bis ungefähr 70°C durchgeführt, solange der Absolutdruck ungefähr 1 bis 1,5 x 10⁵ Pa beträgt, wobei bei anderen Drücken die Temperatur entsprechend modifiziert werden muß. Der letztgenannte Temperaturbereich ist dann bevorzugt, wenn eine selektive Abtrennung der in der ersten Gasphase enthaltenen (Meth)acrylsäure und sonstigen Carbonsäuren von dem dort ebenfalls vorhandenen Alkan, Alkanol, Alken, Alkenal mit 3 oder 4 C-Atomen oder dem Gemisch aus zwei oder mehr davon erreicht werden soll.

Zur Durchführung der Absorption können die erste Gasphase und das Frischwasser im Gleich- oder im Gegenstrom geführt werden, wobei letztere Variante bevorzugt ist. Insbesondere bevorzugt wird die erste Gasphase am unteren Ende der Absorptionsvorrichtung eingebracht, während das Frischwasser am Kopf der Absorptionsvorrichtung eingebracht wird.

Als Absorptionsvorrichtungen kommen insbesondere handelsübliche Absorptionskolonnen in Betracht. Das heißt, die Absorptionskolonne kann z.B. eine Ventilboden-, Glockenboden-, oder sonstige Bodenkolonne, eine Füllkörper- oder eine Packungskolonne sein, die ggf. übliche Einbauten, die genügend Stoff- bzw. Wärmeübertragungsfläche zur Verfügung stellen, aufweisen.

Packungskolonnen sind bevorzugt, wobei unter diesen Packungskolonnen mit geordneten Packungen besonders vorteilhaft sind. Packungskolonnen mit geordneten Packungen sind dem Fachmann an sich bekannt und z.B. in Chem. Ing. Tech. 58 (1986) Nr. 1, S. 19 - 31 beschrieben. Erfindungsgemäß hat sich die Anwendung von Blech- und Plattenpackung als günstig erwiesen. Geordnete Packungen zeichnen sich insbesondere durch einen geringen Druckverlust aus und gewährleisten geringe Verweilzeiten, was im Hinblick auf die hohe Polymerisationsneigung der (Meth)acrylsäure von Vorteil ist.

Ferner umfaßt das erfindungsgemäße Verfahren als Stufe D die Extraktion der zweiten wäßrigen Lösung, die (Meth)acrylsäure enthält, durch Inkontaktbringen mit einer Lösung, die mindestens ein Alkan, Alkanol, Alken, oder Alkenal mit 3 oder 4 C-Atomen oder ein Gemisch aus zwei oder mehr davon enthält, und mit der zweiten wäßrigen Lösung eine Mischungslücke bildet, wobei eine organische Phase, die (Meth)acrylsäure und den Hauptteil an Alkan, Alkanol, Alken Alkenal mit 3 oder 4 C-Atomen oder des Gemischs aus zwei oder mehr davon enthält, und eine dritte wäßrige Lösung, die einen geringeren Teil an Alkan, Alkanol, Alken, Alkenal mit 3 oder 4 C-Atomen oder des Gemischs aus zwei oder mehr davon enthält, erhalten wird.

Wie sich aus obigem ergibt, kann die Extraktion gemäß Stufe D mittels einer Lösung, die ein Alkan, Alkanol, Alken, oder Alkenal mit 3 oder 4 C-Atomen oder ein Gemisch aus zwei oder mehr davon enthält und ferner eine Mischungslücke mit der zweiten wäßrigen Lösung bildet, durchgeführt werden. Dabei wird vorzugsweise die Extraktion gemäß Stufe D mit einer Lösung der gleichen Substanz(en) durchgeführt, die in Stufe A zur Herstellung eines Reaktionsgases, das (Meth)acrylsäure enthält, verwendet wurde(n). Zusätzlich muß für die Verwendung in Stufe D die obige Lösung noch die Eigenschaft aufweisen, daß sie eine Mischungslücke mit der zweiten wäßrigen Lösung aufweist.

Der oben verwendete Begriff "Hauptteil" bedeutet, daß, bezogen auf die Gesamtmenge des der Stufe D zugeführten Alkans, Alkanols, Alkens oder Alkenats mit 3 oder 4 C-Atomen oder des Gemischs aus zwei oder mehr davon, über 50 Gew.-% dieser Gesamtmenge in der organischen Phase sind. Demgemäß bedeutet "geringerer Teil", daß, bezogen auf die Gesamtmenge des der Stufe D zugeführten Alkans, Alkanols, Alkens oder Alkenats mit 3 oder 4 C-Atomen oder des Gemischs aus zwei oder mehr davon, weniger als 50 Gew.-% in der dritten wäßrigen Lösung sind.

Als Substanzen die dafür in Frage kommen, sind vorzugsweise (Meth)acrolein, Isobuten, Propen, Propan, Butan, Isobutyraldehyd, der Methylether des tert.-Butanols (MTBE) oder ein Gemisch aus zwei oder mehr davon zu nennen.

Die Lösung, die mindestens ein Alkan, Alkanol, Alken, Alkenal mit 3 oder 4 C-Atomen oder ein Gemisch aus zwei oder mehr davon enthält, das in (Meth)acrylsäure umgewandelt werden kann, besteht entweder vollständig aus dem Alkan, Alkanol, Alken, Alkenal mit 3 oder 4 C-Atomen oder dem Gemisch aus zwei oder mehr davon oder enthält diese(s) als Gemisch mit anderen Stoffen, wie z.B. Wasser und/oder Essigsäure. Die Lösung kann dabei auch durch den Herstellungsprozeß der Komponenten bedingte Verunreinigungen aufweisen, muß also vor der Extraktion nicht gereinigt werden.

Diese Lösung kann auch mit die Extraktionswirkung verbessernden Zusatzstoffen versetzt werden. Zu nennen sind dabei beispielsweise Entschäumer, wie z.B. Talkfettalkohol und andere Polyalkohole, Deemulgatoren, wie z.B. Alkalimetallchloride und Tenside und Stoffe, die die Mischungslücke erweitern, wie z.B. höhere Alkane, insbesondere solche mit 4 bis 14 C-Atomen.

Bzgl. der Konzentration des in dieser Lösung enthaltenen Alkans, Alkanols, Alkens, Alkenals mit 3 oder 4 C-Atomen oder des Gemischs aus zwei oder mehr davon, das in Meth(acrylsäure) umgewandelt werden kann, bestehen prinzipiell keine Beschränkungen, wobei jedoch die Konzentration dieser Komponente(n) in der Lösung vorzugsweise in einem Bereich von ungefähr 50 bis 100 Gew.-%, weiter bevorzugt ungefähr 70 bis ungefähr 99,9 Gew.-% und insbesondere ungefähr 90 bis ungefähr 97 Gew.-% beträgt.

Dabei muß die oben definierte Lösung in jedem Fall eine Mischungslücke mit der wäßrigen Lösung, die (Meth)acrylsäure enthält, aufweisen.

Im Rahmen des erfindungsgemäßen Verfahrens ist es bevorzugt, zur Extraktion von (Meth)acrylsäure eine Lösung aus Alkan, Alkanol, Alken, oder Alkenal mit 3 oder 4 C-Atomen oder ein Gemisch aus zwei oder mehr davon zu verwenden, die als Edukt für die (Meth)acrylsäure-Synthese eingesetzt werden kann und vorzugsweise eine Konzentration von ungefähr 50 bis 100 Gew.-%, weiter bevorzugt ungefähr 70 bis ungfähr 99,9 Gew.-% und insbesondere ungefähr 90 bis ungefähr 97 Gew.% eines Alkans, Alkanols, Alkens oder Alkenals mit 3 oder 4 C-Atomen oder einem Gemisch aus zwei oder mehr davon enthält. Besonders bevorzugt wird (Meth)acrolein eingesetzt.

Das in dieser Lösung enthaltene Alkan, Alkanol, Alken, Alkenal mit 3 oder 4 C-Atomen oder dem Gemisch aus zwei oder mehr davon, das sich nach der Extraktion zu einem bestimmten Teil in der dritten wäßrigen Lösung befindet, wird vorzugsweise durch ein thermisches Trennverfähren zurückgewonnen, z.B. durch Strippung mit Wasserdampf oder einem Inertgas, wie z.B. Stickstoff, Luft, Kohlendioxid, Abgas aus der (Meth)acrylsäure-Herstellung oder einem Gemisch aus zwei oder mehr davon, oder durch destillative Rückgewinnung. Dabei wird das Alkan, Alkanol, Alken oder Alkenal mit 3 oder 4 C-Atomen oder das Gemisch aus zwei oder mehr davon nahezu vollständig in die Gasphase überführt und kann dann, gegebenenfalls nach Durchlaufen weiterer Stufen, zur Herstellung von (Meth)acrylsäure gemäß Stufe A verwendet werden.

Das bei der Durchführung der thermischen Trennverfahren, z.B. der Strippung der dritten wäßrigen Lösung erhaltene Abwasser enthält lediglich Spuren an weiteren Bestandteilen, wie z.B. Citraconsäuren oder Essigsäure und kann problemlos verworfen werden.

Bezüglich der Konzentration der (Meth)acrylsäure in der zweiten wäßrigen Lösung, die (Meth)acrylsäure enthält, bestehen ebenfalls keinerlei Beschränkungen. Vorzugsweise liegt der Gehalt an (Meth)acrylsäure in dieser Lösung bei ungefähr 1 bis ungefähr 80 Gew.-%, weiter bevorzugt ungefähr 5 bis ungefähr 40 Gew.-% und insbesondere ungefähr 10 bis ungefähr 20 Gew.-%. Neben (Meth)acrylsäure und Wasser enthält diese Lösung eine geringe Menge, in der Regel weniger als ungefähr 3 Gew.-%, des Alkans, Alkanols, Alkens oder Alkenals mit 3 oder 4 C-Atomen oder das Gemisch aus zwei oder mehr davon und eine geringe Menge, in der Regel weniger als 10 Gew.-%, Essigsäure.

Wie oben ausgeführt, wird bei der Extraktion eine organische Phase erhalten, die (Meth)acrylsäure enthält. Dabei wird die Extraktion vorzugsweise so ausgeführt, daß der Hauptanteil der eingesetzten (Meth)acrylsäure sich in dieser Phase befindet. Neben (Meth)acrylsäure enthält die organische Phase noch geringe Mengen an Wasser, Essigsäure und hochsiedenden Bestandteilen, sowie den Hauptteil des Alkans, Alkanols, Alkens oder Alkenals mit 3 oder 4 C-Atomen oder des Gemischs aus zwei oder mehr davon. Die Konzentration an (Meth)acrylsäure in dieser organischen Phase beträgt im allgemeinen ungefähr 1 bis ungefähr 80 Gew.-%, vorzugsweise ungefähr 5 bis ungefähr 40 Gew.-% und insbesondere ungefähr 10 bis ungefähr 30 Gew.-%.

Bezüglich der Temperatur, bei der die Stufe D des erfindungsgemäßen Verfahrens durchgeführt werden kann, existieren keinerlei besondere Beschränkungen. Die einzige Voraussetzung ist, daß es bei der gewählten Temperatur und dem gewählten Druck möglich ist, daß sich zwei Phasen, eine organische und eine wäßrige Phase, ausbilden. Im allgemeinen wird die Extraktion gemäß Stufe D bei einer Temperatur von ungefähr 0 bis ungefähr 150°C, vorzugsweise ungefähr 30 bis ungefähr 80°C und insbesondere ungefähr 50 bis ungefähr 70°C durchgeführt, wobei bei Temperaturen oberhalb des Siedepunktes des Alkans, Alkanols, Alkens oder Alkenals mit 3 oder 4 C-Atomen oder das Gemisch aus zwei oder mehr davon als Extraktionsmittels bei überatmosphärischem Druck gearbeitet wird. Bei Verwendung von (Meth)acrolein als Extraktionsmittel wird bei Temperaturen oberhalb von ungefähr 68°C bei überatmosphärischen Druck gearbeitet. Bei der Verwendung einer anderen Alkans, Alkanols, Alkens, oder Alkenats mit 3 oder 4 C-Atomen oder einem Gemisch aus zwei oder mehr davon müssen Temperatur und/oder Druck in Abhängigkeit von diesem modifiziert werden.

Zur Durchführung der Extraktion können alle Vorrichtungen, die im allgemeinen zur Extraktion benutzt werden, wie sie beispielsweise in Ullmann-Enzyclopädie der Technischen Chemie, 4. Auflage, Bd. 2, S. 546 ff, insbesondere S. 560 ff (1972), beschrieben sind. Zu nennen sind insbesondere Mixer-Settler-Vorrichtungen, Extraktions-Kolonnen, Sprühturm-Extraktionskolonnen, pulsierte bzw. unpulsierte Boden-, Füllkörper- Extraktionsvorrichtungen, die Zentrifugalkräfte nutzen.

Dabei können die wäßrige Lösung, die (Meth)acrylsäure enthält, und die Lösung, die mindestens ein Alkan, Alkanol, Alken, oder Alkenal mit 3 oder 4 C-Atomen oder ein Gemisch aus zwei oder mehr davon enthält, entweder im Gleichstrom, Kreuzstrom oder im Gegenstrom miteinander in Kontakt gebracht werden, wobei das Inkontaktbringen im Gegenstrom bevorzugt ist.

Die Extraktion kann ein- oder mehrstufig durchgeführt werden, wobei auch Kombinationen von Extraktionsvorrichtungen verwendet werden können.

Die Extraktion gemäß Stufe D kann kontinuierlich oder diskontinuierlich durchgeführt werden.

Wie oben erwähnt wird das zur Extraktion verwendete, sich in geringer Menge in der dritten wäßrigen Losung befindliche Alkan, Alkanol, Alken oder Alkenal mit 3 oder 4 C-Atomen oder das Gemisch aus zwei oder mehr davon nach Durchlaufen der Extraktion vorzugsweise aus dieser Lösung zurückgewonnen, wobei ein im wesentlich von (Meth)acrylsäure und freies Abwasser und eine sechste Gasphase, die Alkan, Alkanol, Alken, Alkenal mit 3 oder 4 C-Atomen oder ein Gemisch aus zwei oder mehr davon enthält, erhalten wird. Als Methode zur Rückgewinnung des Alkans, Alkanols, Alkens, oder Alkenats mit 3 oder 4 C-Atomen oder des Gemischs aus zwei oder mehr davon eignen sich prinzipiell alle thermischen Trennverfahren, wobei insbesondere das Strippen mit einem Inertgas, wie z.B. Stickstoff, Luft, Kohlendioxid, einem Abgas, das bei der Herrstellung von (Meth)acrylsäure entsteht, oder eines Gemischs dieser Inertgase, bevorzugt ist. Besonders bevorzugt ist es, die für die Oxidation benötigte Luft zu verwenden.

Der Strippvorgang wird im allgemeinen bei einer Temperatur von ungefähr 30 bis ungefähr 100°C, vorzugsweise ungefähr 50 bis ungefähr 80°C durchgeführt. Dies gilt für einen Absolutdruck von 1 bar und ist für andere Drücke zu modifizieren.

Gasmenge, Temperatur und Druck wird im allgemeinen so eingestellt, daß eine vollständige Rückgewinnung des Alkans, Alkanols, Alkens, oder Alkenats mit 3 oder 4 C-Atomen oder des Gemischs aus zwei oder mehr davon erreicht wird.

Die Art der verwendeten Strippvorrichtung unterliegt keiner bestimmten Beschränkung, und es können beliebige herkömmliche Strippvorrichtungen verwendet werden, die einen Gas-Flüssigkeits-Kontakt ermöglichen, z.B. Füllkörper-, Siebboden-, Glockenboden- oder anderer Bodenkolonnen, Packungskolonnen oder Sprühtürme. Weitere gut verwendbare Sprühvorrichtungen sind unter dem Stichwort "Absorptionskolonne" in der EP-A 706 986 in Spalte 3, Zeilen 11 bis 38 und dem darin zitierten Stand der Technik beschrieben, der durch Bezugnahme vollumfänglich in die vorliegende Anmeldung mit einbezogen wird.

Wie oben ausgeführt, wird die in der organischen Phase enthaltene (Meth)acrylsäure ebenfalls zurückgewonnen, z.B. indem man die in Stufe D erhaltene organische Phase der Kondensationsstufe B zuführt. Innerhalb dieser Stufe wird die Hauptmenge an Alkan, Alkanol, Alken, Alkenal mit 3 oder 4 C-Atomen oder des Gemischs aus zwei oder mehr davon in den gasförmigen Zustand überführt und die Hauptmenge an (Meth)acrylsäure verbleibt in der ersten wäßrigen Lösung.

Ferner ist es möglich, die organische Phase, die (Meth)acrylsäure enthält, thermisch, z.B. durch Destillation/Rektifikation aufzutrennen, vorzugsweise unter Ausnutzung der Energie der Reaktionsgase, wobei eine Fraktion, die (Meth)acrylsäure enthält, und eine Fraktion, die das das Alkan, Alkanol, Alken, oder Alkenal mit 3 oder 4 C-Atomen oder das Gemisch aus zwei oder mehr davon enthält, erhalten wird.

In einer weiteren Ausführungsform der vorliegenden Erfindung wird die erste wäßrige Phase aus Stufe B, die die Hauptmenge an im Reaktionsgas enthaltener (Meth)acrylsäure und eine Restmenge an im Reaktionsgas enthaltenem Alkan, Alkanol, Alken, Alkenal mit 3 oder 4 C-Atomen oder des Gemischs aus zwei oder mehr davon enthält, durch ein thermisches Trennverfahren vom Alkan, Alkanol, Alken, Alkenal mit 3 oder 4 C-Atomen oder dem Gemisch aus zwei oder mehr davon befreit oder mit einem Inertgas in Kontakt gebracht, d.h. gestrippt, wobei das darin enthaltene Alkan, Alkanol, Alken, Alkenal mit 3 oder 4 C-Atomen oder das Gemischs aus zwei oder mehr davon zusammen mit einem Teil des enthaltenen Wassers als dritte Gasphase entfernt wird, wobei man eine wäßrige (Meth)acrylsäure-Lösung mit einem Gehalt an (Meth)acrylsäure von bis zu 100 Gew.-%, vorzugsweise ungefähr 50 bis ungefähr 90 Gew.-%, erhält.

Zur Durchführung dieser Teilstufe des erfindungsgemäßen Verfahrens als Strippung wird die erste wäßrige Phase in eine Strippvorrichtung im mittleren Teil oder am oberen Ende, vorzugsweise am oberen Ende derselben, eingeleitet. Ein Inertgas, wie z.B. Stickstoff, Luft, Kohlendioxid, Abgas, das bei der Gasphasenoxidation im Rahmen der Herstellungsstufe A entsteht, Abgas, das bei der Verbrennung des erstgenannten Abgases entsteht, oder ein Gemisch dieser Inertgase, wird am vorzugsweise unteren Teil des Turms eingeleitet, wobei Gasmenge, Temperatur und Druck vorzugsweise so eingestellt werden, daß ein Großteil des vorhandenen Alkans, Alkanols, Alkens oder Alkenals mit 3 oder 4 C-Atomen oder des Gemischs aus zwei oder mehr davon abgetrennt wird.

Die Betriebstemperatur der verwendeten Strippvorrichtung beträgt im allgemeinen ungefähr 150°C oder weniger, vorzugsweise ungefähr 30 bis ungefähr 150°C und insbesondere ungefähr 80 bis ungefähr 130°C, jeweils bei einem Druck von ungefähr 1 bis ungefähr 1,5 x 10⁵ Pa, wobei auch hier bei der Wähl anderer Temperaturen entsprechend modifiziert werden muß.

Die Art der Strippvorrichtung unterliegt keiner bestimmten Beschränkung und es können beliebige herkömmliche Strippvorrichtungen, vorzugsweise Stripptürme, verwendet werden, die einen möglichst guten Gas-Flüssigkeits-Kontakt ermöglichen, wie z.B. Füllkörper, Siebboden-, Glockenboden- oder Sprühtürme. Bezüglich weiterer Details wird auf "Ullmann's Enzyklopädie der Technischen Chemie", Bd. 2, S. 575 ff., insbesondere S. 587/8 und 589 ff. (1972) verwiesen.

Im Rahmen dieser Teilstufe wird das in der ersten wäßrigen Lösung enthaltene Alkan, Alkanol, Alken, Alkenal mit 3 oder 4 C-Atomen oder das Gemisch aus zwei oder mehr davon praktisch vollständig abgetrennt und fällt am oberen Ende der Strippvorrichtung in Form eines Gasgemisches an (dritte Gasphase), während am unteren Ende der Vorrichtung eine wie oben definierte wäßrige (Meth)acrylsäure-Lösung erhalten wird.

Die so erhaltene wäßrige (Meth)acrylsäure-Lösung enthält gegebenenfalls noch geringe Mengen (unter 20 Gew.-%) Essigsäure und (unter 10 Gew.-%) hochsiedende Bestandteile.

Die Essigsäure und die hochsiedenden Bestandteile können bei der weiteren Aufarbeitung der erhaltenen wäßrigen (Meth)acrylsäure-Lösung nach an sich bekannten Verfahren, wie sie beispielsweise in den DE-OS 43 35 172 und DE-OS 42 01 697 entfernt werden.

Insbesondere erfolgt die Aufarbeitung der erhaltenen wässrigen (Meth)acrylsäure-Losung durch Kristallisation. Eine derartige Kristallisation ist u.a. in der DE-A 197 40 252.6 beschrieben, die vollumfänglich in dem Kontext der vorliegenden Anmeldung einbezogen wird.

Im folgenden soll dennoch die erfindungsgemäß ggf. durchgeführte Kristallisation unter Bezugnahme auf Fig. 3 in ihren Grundzügen erläutert werden.

Erfindungsgemäß kann ein Teil oder die gesamte Menge der kondensierten MAS-Lösung (B1) bzw. der in der Methacrolein-Abtrennung (MA), in der auch die Schwersieder (SS) abgetrennt werden können, erhaltenen Lösung (MA1) einer Reinigung durch Kristallisation unterzogen werden.

Dabei wird in der Kristallisation und Fest-Flüssig-Trennung (KR + FFT) ein (Meth)acrylsäure-Kristallisat (KR1) gewonnen, das abhängig von der Reinheit als reine (Meth)acrylsäure oder zur Herstellung von (Meth)acrylsäureestern verwendet werden kann.

Die aus der Stufe KR + FFT kommende Mutterlauge enthält neben Wasser und (Meth)acrylsäure Nebenprodukte wie z.B. Essigsäure. Diese Mutterlauge kann ganz oder teilweise aus dem Verfahren ausgeschleust werden (ML1); zur Rückgewinnung von (Meth)acrylsäure kann die Mutterlauge komplett oder zum Teil in die (Meth)acrylsäure-Extraktion gemäß Stufe D (ML2) und/oder in die Stufe B (ML3) und/oder in die Methacrolein-Abtrennung (ML4) gefahren werden.

Im einzelnen wird dabei wie folgt vorgegangen:

In Stufe (KR + FFT) wird die in Stufe (B) oder (MA) erhaltene Lösung, die (Meth)acrylsäure enthält, kristallisiert. Hierbei wird ohne Zusatz eines Lösungsmittels, insbesondere ohne Zusatz eines organischen Lösungsmittels, gearbeitet. Das verwendete Kristallisationsverfahren unterliegt keiner Beschränkung. Die Kristallisation kann kontinuierlich oder diskontinuierlich, einstufig oder mehrstufig durchgeführt werden. Vorzugsweise erfolgt die Kristallisation einstufig. In einer anderen bevorzugten Ausführungsform wird die Kristallisation als fraktionierte Kristallisation durchgeführt. Üblicherweise werden bei fraktionierter Kristallisation alle Stufen, die ein Kristallisat erzeugen, das reiner ist als die zugeführte wäßrige (Meth)acrylsäurelösung, Reinigungsstufen genannt und alle anderen Stufen Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt wird und dieses Kristallisat der jeweiligen Stufe mit dem nächsthöheren Reinbeitsgrad zugeführt wird, während der Kristallisationsrückstand der jeweiligen Stufe mit dem nächstniedrigen Reinheitsgrad zugeführt wird.

Vorteilhafterweise liegt die Temperatur der Lösung während der Kristallisation zwischen -25°C und +15°C, insbesondere zwischen -15°C und 5°C. Der Feststoffgehalt im Kristallisator beträgt vorteilhafterweise 0 bis 90 g/100 g, bevorzugt 15 bis 65 g Feststoff/100 g.

In einer weiteren vorteilhaften Ausgestaltung erfolgt die Kristallisation durch Kühlung von Apparatewänden oder durch partielle Verdampfung der Lösung im Vakuum. Bei der Kristallisation durch Kühlung wird die Wärme über Kratzkühler, die mit einem Rührkessel oder einem Behälter ohne Rührwerk verbunden sein können, abgeführt. Der Umlauf der Kristallsuspension wird hierbei durch eine Pumpe gewährleistet. Daneben besteht auch die Möglichkeit, die Wärme über die Wand eines Rührkessels, insbesondere mit wandgängigem Rührer, abzuführen. Eine weitere bevorzugte Ausführungsform bei der Kühlungskristallisation ist die Verwendung von Kühlscheibenkristallisatoren, wie sie z.B. von der Fa. GMF Gouda (Holland) hergestellt werden. Bei einer weiteren geeigneten Variante zur Kristallisation durch Kühlung wird die Wärme über herkömmliche Wärmeüberträger (bevorzugt Rohrbündel- oder Plattenwärmeüberträger) abgeführt. Diese Apparate besitzen im Gegensatz zu Kratzkühlern, Rührkesseln mit wandgängigen Rührern oder Kühlscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche partielle Verdampfung der Lösung im Vakuum erfolgen. In einer weiteren vorteilhaften Ausführung der Erfindung erfolgt die Kristallisation in Apparaten, in denen die Kristalle im Kristallisationsapparat an gekühlten Flächen aufwachsen, d.h. im Apparat fixiert sind (z.B. Schichtkristallisationsverfahren der Fa. Sulzer Chemtech (Schweiz) oder Statisches Kristallisationsverfahren der Fa. BEFS PROKEM (Frankreich)).

Die in Stufe (KR + FFT) erhaltenen (Meth)acrylsäurekristalle werden von der Mutterlauge abgetrennt. Für den Fall der Schichtkristallisation oder der Statischen Kristallisation kann die Trennung der Kristalle von der Mutterlauge im Kristallisationsapparat selbst erfolgen, da die Kristalle im Apparat fixiert sind und die Mutterlauge durch Abfließenlassen aus dem Apparat entfernt werden kann. Die Entfernung der Kristalle aus dem Kristallisationsapparat erfolgt durch Aufschmelzen der Kristalle und nachfolgendes Abfließenlassen der Schmelze. Für den Fall der Suspensionskristallisation eignen sich alle bekannten Verfahren der Fest-Flüssig-Trennung. In einer bevorzugten Ausführungsform der Erfindung werden die Kristalle durch Filtrieren und/oder Zentrifugieren von der Mutterlauge abgetrennt. Vorteilhafterweise wird dem Filtrieren oder Zentrifugieren eine Voreindickung der Suspension, zum Beispiel durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk betrieben werden oder mittels Bandfilter. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an. Beim Waschen liegt die Waschflüssigkeitsmenge geeigneterweise zwischen 0 und 500 g Waschflüssigkeit/100 g Kristallisat, vorzugsweise zwischen 30 und 200 g Waschflüssigkeit/100 g Kristallisat. Die verwendete Waschflüssigkeit unterliegt keiner Einschränkung. Vorteilhafterweise wird jedoch mit Reinprodukt gewaschen, d.h. mit einer Flüssigkeit, die (Meth)acrylsäure enthält, deren Reinheit in der Regel höher ist als die des zu waschenden Kristallkuchens, mindestens aber reiner ist als die Mutterlauge in der Kristallisation. Daneben ist auch eine Wäsche mit Wasser möglich. Das Waschen kann in hierfür üblichen Apparaten erfolgen. Vorteilhafterweise werden Waschkolonnen, in denen die Abtrennung der Mutterlauge und das Waschen in einem Apparat erfolgen, Zentrifugen, die ein- oder mehrstufig betrieben werden können, oder Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Beim Schwitzen handelt es sich um ein lokales Abschmelzen verunreinigter Bereiche. Vorteilhafterweise beträgt die Schwitzmenge zwischen 0 und 100 g abgeschmolzenes Kristallisat/100 g Kristallisat vor dem Schwitzen, vorzugsweise zwischen 5 und 35 g abgeschmolzenes Kristallisat/100 g Kristallisat. Besonders bevorzugt ist die Durchführung des Schwitzens auf Zentrifugen oder Bandfiltern. Auch die Durchführung einer Kombination aus Waschen und Schwitzen in einem Apparat kann geeignet sein.

Die während des Strippens erhaltene dritte Gasphase, die Alkan, Alkanol, Alken oder Alkenal mit 3 oder 4 C-Atomen oder ein Gemisch aus zwei oder mehr davon und Wasser enthält, kann im Rahmen des erfindungsgemäßen Verfahrens in die Kondensationsstufe B und/oder die Absorptionsstufe C eingebracht und dort weiter aufgearbeitet werden.

Gemäß einer weiteren Ausführungsform der vorliegenden Erfindung wird die erste Gasphase alleine oder zusammen mit der dritten Gasphase vor der Absorption gemäß Stufe C in einen Kreisgasstrom, der in die Absorptionsstufe C eingebracht wird, und einen Abgasstrom aufgeteilt.

Der Abgasstrom kann in einer weiteren zusätzlichen Stufe E einer Kondensation unterzogen werden, wobei eine vierte wäßrige Lösung, die die Hauptmenge an (Meth)acrylsäure und eine Restmenge an Alkan, Alkanol, Alken, Alkenal mit 3 oder 4 C-Atomen oder dem Gemisch aus zwei oder mehr davon aus dem Abgasstrom enthält, und eine vierte Gasphase, die die Hauptmenge an Alkan, Alkanol, Alken, Alkenal mit 3 oder 4 C-Atomen oder dem Gemisch aus zwei oder mehr davon und eine Restmenge an (Meth)acrylsäure aus dem Abgasstrom enthält, erhalten wird.

Die Verfahrensbedingungen der Stufe E sowie die dort verwendeten Vorrichtungen entsprechen den bezüglich der Stufe B bereits genannten und diskutierten Bedingungen bzw. Vorrichtungen.

Bezüglich der für diese Kondensation verwendeten Temperaturen trifft auch das bezüglich der Stufe B Gesagte zu, wobei jedoch bei der Kondensation gemäß Stufe E tendenziell etwas niedrigere Temperaturen verwendet werden, so daß der für diese Stufe bevorzugte Temperaturbereich bei ungefähr 30 bis ungefähr 50°C liegt, jeweils bei einem Druck von ungefähr 1 bis ungefähr 1,5 x 10⁵ Pa, wobei auch hier bei der Wahl anderer Temperaturen entsprechend modifiziert werden muß.

Die in der Stufe E erhaltene vierte Gasphase kann gemäß einer weiteren Ausführungsform der vorliegenden Erfindung einer Absorptionsstufe F unterworfen werden, in der das in der vierten Gasphase enthaltene Alkan, Alkanol, Alken, oder Alkenal mit 3 oder 4 C-Atomen oder das Gemisch aus zwei oder mehr davon durch Inkontaktbringen mit einer fünften wäßrigen Lösung, die (Meth)acrylsäure enthält, nahezu vollständig aus der vierten Gasphase entfernt wird, wobei ein Abgas, das im wesentlichen frei ist von (Meth)acrylsäure, Alkan, Alkanol, Alken Alkenal mit 3 oder 4 C-Atomen oder dem Gemisch aus zwei oder mehr davon, und eine mit Alkan, Alkanol, Alken Alkenal mit 3 oder 4 C-Atomen oder dem Gemisch aus zwei oder mehr davon beladene Kreislauflösung, die (Meth)acrylsäure enthält, erhalten wird.

Die Absorptionsstufe F wird im wesentlichen entsprechend der Absorptionsstufe C durchgeführt, wobei zu berücksichtigen ist, daß die bei der Absorptionsstufe F verwendeten Temperaturen - verglichen mit den für die Absorptionsstufe C angegebenen Temperaturen - im allgemeinen niedriger liegen und die Absorption gemäß Stufe F im allgemeinen bei ungefähr 0 bis ungefähr 50°C und vorzugsweise bei ungefähr 4 bis ungefähr 30°C durchgeführt wird, jeweils bei einem Druck von ungefähr 1 bis ungefähr 1,5 x 10⁵ Pa, wobei auch hier bei der Wahl anderer Temperaturen entsprechend modifiziert werden muß.

Das in der Stufe F erhaltene Abgas kann gegebenenfalls zur Strippung des Alkans, Alkanols, Alkens, oder Alkenals mit 3 oder 4 C-Atomen oder des Gemischs aus zwei oder mehr davon an verschiedenen Stellen des erfindungsgemäßen Verfahrens verwendet werden, oder aber - vollständig oder partiell - wieder in die Herstellungsstufe A eingespeist werden.

Das in der beladenen Kreislautlösung enthaltene Alkan, Alkanol, Alken oder Alkenal mit 3 oder 4 C-Atomen oder das Gemisch aus zwei oder mehr davon kann durch Inkontaktbringen mit einem Gas, das ein Alkan, Alkanol, Alken, Alkenal mit 3 oder 4 C-Atomen oder ein Gemisch aus zwei oder mehr davon enthält, vorzugsweise mit dem Kreisgasstrom, desorbiert werden, wobei eine sechste wäßrige Lösung, die (Meth)acrylsäure enthält und eine fünfte Gasphase, die das Alkan, Alkanol, Alken oder Alkenal mit 3 oder 4 C-Atomen oder das Gemisch aus zwei oder mehr davon enthält, erhalten wird (Stufe G).

Die beladene Kreislauflösung wird dabei in den unteren Teil der Absorptionsvorrichtung zur Durchführung von Stufe C eingeleitet. Selbstverständlich kann die Desorption des Alkans, Alkanols, Alkens, oder Alkenats mit 3 oder 4 C-Atomen oder des Gemischs aus zwei oder mehr davon auch getrennt davon durchgeführt werden, was jedoch nicht bevorzugt ist, da dies einen zusätzlichen apparativen Aufwand bedeutet.

Bezüglich der Durchführung der Desorption gemäß Stufe G wird bezüglich der verwendbaren Vorrichtungen und Temperaturen auf die Absorption gemäß Stufe C verwiesen. Neben der bei der Absorption gemäß Stufe C [(Meth)acrylsäure-Absorption] erhaltenen zweiten wäßrigen Lösung können auch die bei der Kondensation gemäß Stufe E [(Meth)acrylsäure-Kondensation] erhaltene vierte wäßrige Lösung oder die bei der Absorption gemäß Stufe F [Absorption des Alkans, Alkanols, Alkens, oder Alkenals mit 3 oder 4 C-Atomen oder des Gemischs aus zwei oder mehr davon] erhaltene beladene Kreislauflösung, oder die bei der Desorption gemäß Stufe G [Desorption des Alkans, Alkanols, Alkens, oder Alkenals mit 3 oder 4 C-Atomen oder des Gemischs aus zwei oder mehr davon] erhaltene sechste wäßrige Lösung, die jeweils (Meth)acrylsäure enthalten, und einzeln oder als Kombination aus zwei oder mehr davon, ggf. zusammen mit der zweiten wäßrigen Lösung, mindestens einen Zulauf bilden, in die Extraktionsstufe D eingebracht werden. Dabei ist es bevorzugt, daß die oben beschriebenen wäßrigen Lösungen mit der zweiten wäßrigen Lösung zusammen in Stufe D eingebracht werden.

In einer weiteren, bevorzugten Ausführungsform werden die obengenannten wäßrigen Lösungen, die jeweils (Meth)acrylsäure enthalten, vor der Extraktion gemäß Stufe D in einen Strom zur Wasserausschleusung, der in die Extraktionsstufe D eingebracht wird, und eine Kreislauflösung für die Abgaswäsche aufgeteilt. Dabei ist das Verhältnis der Aufteilung Strom zur Wasserausschleusung/Kreislauflösung vorzugsweise so wählen, daß die in Stufe C zugeführte Frischwassermenge im Strom zur Wasserausschleusung enthalten ist. Der Strom zur Wasserausschleusung wird dann der Extraktion gemäß Stufe D, wie oben beschrieben, unterworfen und weiter aufgearbeitet.

Während, wie oben ebenfalls ausgeführt, die organische Phase, die (Meth)acrylsäure enthält, vorzugsweise der Kondensationsstufe B zugeführt wird, wird die dritte wäßrige Lösung, die das Alkan, Alkanol, Alken oder Alkenal mit 3 oder 4 C-Atomen oder das Gemisch aus zwei oder mehr davon enthält, mit einem Inertgas in Kontakt gebracht (Strippung), wobei ein Abwasser, das im wesentlichen frei ist von Alkan, Alkanol, Alken, Alkenal mit 3 oder 4 C-Atomen oder dem Gemisch aus zwei oder mehr davon Edukt und (Meth)acrylsäure und gegebenenfalls lediglich Spuren dieser beiden Komponenten und unter Umständen Essigsäure enthält, und eine sechste Gasphase, die das Alkan, Alkanol, Alken oder Alkenal mit 3 oder 4 C-Atomen oder das Gemisch aus zwei oder mehr davon enthält, erhalten wird. Insbesondere bevorzugt ist die Oxidationsluft als Strippgas. Es kann aber auch eine andere thermische Trennoperation verwendet werden.

Bezüglich der Verfahrensbedingungen und apparativen Voraussetzungen für die Strippung unter Erhalt der sechsten Gasphase, wird auf die vorstehend bezüglich der Entfernung von Alkan, Alkanol, Alken, Alkenal mit 3 oder 4 C-Atomen oder des Gemischs aus zwei oder mehr davon aus der ersten wäßrigen Lösung gemachten Ausführungen, sowie die ausführliche Beschreibung der Stufe D, in der ebenfalls die Aufarbeitung der dritten wäßrigen Lösung diskutiert wird, verwiesen.

Die gegebenenfalls abgetrennte Kreislauflösung für die Abgaswäsche kann ebenfalls durch Inkontaktbringen mit einem Inertgas (Strippung) behandelt werden, insbesondere bevorzugt ist es, einen Teil der Oxidationsluft zu verwenden, wobei eine regenerierte Kreislauflösung und eine siebte Gasphase, die Alkan, Alkanol, Alken oder Alkenal mit 3 oder 4 C-Atomen oder das Gemisch aus zwei oder mehr davon enthält, erhalten wird.

Die Kreislauflösung oder die regenerierte Kreislauflösung werden vorzugsweise als die fünfte wäßrige Lösung, die (Meth)acrylsäure enthält, in die Absorptionsstufe F eingebracht.

Die im Rahmen des vorliegenden Verfahrens an verschiedenen Stellen erhaltenen Gasphasen, also die erste, zweite, dritte, fünfte, sechste und siebte Gasphase, die jeweils Alkan, Alkanol, Alken oder Alkenal mit 3 oder 4 C-Atomen oder das Gemisch aus zwei oder mehr davon enthalten, können entweder einzeln oder als Kombination aus zwei oder mehr davon in die Herstellungsstufe A eingebracht werden, wobei eine vollständige Rückführung dieser Gasphasen bevorzugt wird.

Den organischen Phasen, bzw. wässrigen Lösungen kann selbstverständlich zur Vermeidung bzw. zur Verringerung der Polymerisationsneigung der (Meth)acrylsäure bzw. des Alkans, Alkanols, Alkens, oder Alkenats mit 3 oder 4 C-Atomen oder des Gemischs aus zwei oder mehr davon ein gebräuchlicher Stabilisator, wie z.B. Phenothiazin oder Hydrochinon, oder dessen Derivate zugesetzt werden.

Im folgenden wird nochmals eine besonders bevorzugte Ausführungsform des erfindungsgemäßen Verfahrens zur Herstellung von (Meth)acrylsäure unter Verwendung von Methacrolein als Extraktionsmittel für Methacrylsäure unter Bezugnahme auf Fig. 1 beschrieben, wobei die durchgezogenen Linien flüssige Ströme und die schraffierten Linien gasförmige Ströme bezeichnen:

In einer heterogen-katalysierten Reaktion wird Methacrolein (MAC) mit Luftsauerstoff zu Methacrylsäure (MAS) oxidiert. Als Nebenprodukt bildet sich Essigsäure (ES) (Stufe A).

Das Reaktionsgas mit MAS, ES, MAC, O₂ (Sauerstoff) und H₂O (A1) wird in der Reaktionsgaskühlung (Kondensation gemäß Stufe B) abgekühlt und die Hauptmenge der im Reaktionsgas enthaltenen Methacrylsäure und der Essigsäure partiell kondensiert. Die erhaltene MAS-Lösung (B1) wird der Methacrolein-Abtrennung (MA) zugeführt. In der Methacrolein-Abtrennung wird aus der MAS-Lösung (B1) MAC und Wasser abgetrennt und die Methacrylsäure aufkonzentriert. Das MAC und Wasser enthaltende Gas wird in die Stufe B zurückgeführt (nicht dargestellt).

Das Gas (B) (erste Gasphase) mit der in Stufe B nicht abgetrennten MAS und ES, das die Hauptmenge an MAC enthält, wird in einen Kreisgasstrom (B2) und einen Abgasstrom (B3) aufgeteilt.

Der Kreisgasstrom strippt in der Methacrolein-Desorption (Stufe G) zuerst Methacrolein aus der zur Abgaswäsche verwendeten Kreislauflösung (F1) und gelangt dann in die Methacrylsäure-Absorption (Stufe C).

In der Methacrylsäure-Absorption wird aus dem Kreisgas (B2/G2) und der mit MAC und MAS und ES beladenen Oxidationsluft (MS1/R1), wie untenstehend diskutiert, mit Frischwasser MAS und ES abgetrennt, während MAC im Kreisgas verbleibt. Das mit MAC und Sauerstoff angereicherte Kreisgas (C1) wird wieder der Stufe A zugeführt. Ferner wird eine wäßrige Lösung (C2), die MAS enthält, erhalten.

Der Abgasstrom (B3) wird der Methacrylsäure-Kondensation (Stufe E) zugeführt, wobei ein Großteil der darin vorhandenen MAS und ES auskondensiert (E1). Die bei dieser Kondensation erhaltene Gasphase (E2) wird der Methacrolein-Absorption gemäß Stufe F, die später noch genauer erläutert wird, zugeführt.

Die wäßrigen Lösungen C2, G1 (wird später noch erläutert) und E1 aus der Methacrylsäure-Absorption, der Methacrolein-Desorption und der Methacrylsäure-Kondensation werden in eine Kreislauflösung für die Abgaswäsche (K1) und einen Strom zur Ausschleusung des Wassers (W) aufgeteilt.

Da über die Wasserbilanz die Menge des ausgeschleusten Wassers festgelegt ist und in diesem Strom auch die in der Kondensationsstufe B nicht kondensierte MAS enthalten sein muß, pegelt sich MAS in der Kreislauflösung auf. Abhängig von der zu- bzw. abgeführten Wassermenge läßt sich die MAS-Konzentration in der Kreislauflösung bzw. im Strom zur Ausschleusung des Wassers einstellen.

Die Methacrylsäure und der überwiegende Teil der Essigsäure werden aus dem Strom zur Ausschleusung des Wassers (W) in der MethacrylsäureExtraktion (Stufe D) mit der als Edukt für die MAS-Herstellung verwendeten Methacrolein-Lösung (M) abgetrennt, wobei eine organische Phase (D1), die die MAS und die Hauptmenge der Essigsäure, die jeweils in dem Strom zur Ausschleusung des Wassers vorhanden sind, sowie den Hauptteil an MAC enthält, und eine wäßrige Phase (D2), die einen geringeren Teil an MAC enthält, erhalten wird.

Das in der wäßrigen Phase (D2) gelöste MAC wird in der Methacrolein-Strippung (MS) durch Desorption mit einem Teil der für die Oxidation benötigten Luft zurückgewonnen.

Die organische Phase (D1), die MAS, ES und MAC enthält, wird in die Reaktionsgas-Kühlung (B) eingebracht und dort aufgetrennt. Während MAC verdampft und damit die Kühlleistung vermindert, bleibt MAS in der flüssigen Phase und erhöht die Konzentration der aus der Reaktionsgas-Kühlung abgetrennten MAS-Lösung (B1).

Aus der Kreislauflösung für die Abgaswäsche (K1) wird in der Regeneration (R) MAC nahezu vollständig abgetrennt. Die Abtrennung wird durch Strippung mit einem Teil der für die Oxidation benötigten Luft erreicht.

Die Oxidationsluft aus der Methacrolein-Strippung des Ausschleusestroms (MS1) und aus der Regenerierung der Kreislauflösung (R1) wird mit dem die Methacrolein-Resorption gemäß Stufe G durchlaufenden Kreisgas (G2) in die Methacrylsäure-Absorption (Stufe C) gefahren.

Die regenerierte Kreislauflösung (K2) wird abgekühlt und zur MAC-Absorption (Stufe F) aus dem Abgasstrom (B3/E2) verwendet. Durch den MAS-Gehalt in der regenierten Kreislauflösung (K2) wird die MAC-Löslichkeit im Vergleich zu reinem Wasser verbessert und damit die Methacrolein-Absorption erleichtert. Nach der Methacrolein-Desorption gemäß Stufe G mit dem Kreisgas (B2) wird die danach erhaltene wäßrige Lösung (G1) wieder in die Regenerierung/Extraktion geführt.

In Fig. 1 bedeuten ferner:
AG - Abgas
FW - Frischwasser
KR - Kristallisation (s. Fig. 3)
L - Luft
MAS-MAC - Methacrylsäurelösung ohne Methacrolein
WW - Abwasser

### BEISPIEL

Im folgenden wird unter Bezugnahme auf Fig. 2 ein Ausführungsbeispiel des erfindungsgemäßen Verfahrens beschrieben, wobei die Zusammensetzung sowie die Menge, Dichte und Temperatur der im folgenden beschriebenen flüssigen und gasförmigen Ströme in der beiliegenden Tabelle aufgeführt sind.

In einer heterogen-katalysierten Oxidation wurde Methacrolein (MAC) zu Methacrylsäure (MAS) unter Bildung von Essigsäure (ES) als Nebenprodukt im Reaktor (R) oxidiert. Das ungefähr 220°C heiße Reaktionsgas (4) wurde in der Quenchkolonne K 1000 (Durchmesser 100 mm, 1000 mm Pall-Ring-Schüttung) auf ungefähr 63 °C abgekühlt. Dabei wurden aus ungefähr 30kg/h Reaktionsgas ungefähr 70 Gew.-% der Methacrylsäure und 44 Gew.-% der Essigsäure, jeweils bezogen auf die im Reaktionsgas vorhandene Gesamtmenge dieser beiden Komponenten, auskondensiert. Die Kühlung des Reaktionsgases erfolgte dabei einerseits über den Kühler W 1100 im Flüssigkeitskreislauf (6a, 6) und andererseits durch Verdampfung von Methacrolein und Wasser in der Quenchkolonne K 1000.

Um Polymerisation in der Quenchkolonne K 1000 zu verhindern, wurde Hydrochinon als Stabilisator in den Flüssigkeitskreislauf (6, 6a) dosiert.

Das in der ersten wässrigen Lösung aus der Quenchkolonne K 1000 noch enthaltene MAC wurde im MAC-Stripper K 1100 (Durchmesser 30 mm, 500 mm-Sulzer Mellapack) abgetrennt und eine 80 gew.-%ige Methacrylsäurelösung erhalten (5).

Der in der Quenchkolonne K 1000 nicht kondensierte Teil des Reaktionsgases mit der dort verbliebenene MAS und ES (5a) wurde zum überwiegenden Teil in der Absorptionskolonne K 2000 (Durchmesser 100mm, 3000 mm Pall-Ring-Schüttung) mit einer auf bis zu 5 kg/h reduzierten Frischwassermenge (20) fast vollständig abgetrennt, während das Edukt Methacrolein im Kreisgas (2) blieb. Durch begrenzte Abkühlung der Quenchkolonne K 1000 und temperierte Frischwasserzugabe wurde am Kopf der Absorptionskolonne K 2000 ein Wassergehalt von 8,7 Gew-% im Kreisgas eingehalten.

Im Sumpf der Absorptionskolonne K 2000 wurde das Abgas (12) aus dem Kreisgasstrom zur Abgaskolonne K 3000 ausgeschleust.

Die aus der Absorptionskolonne K 2000 ablaufende wäßrige Lösung wurde in einen Kreislaufstrom (7/7a) von ungefähr 17,5 kg/h zur Abgaswäsche und einen Strom zur Ausschleusung von Wasser (21) von ungefähr 6,1 kg/h aufgeteilt. Da über die Wasserbilanz die Menge des Ausschleusestroms festgelegt war und in diesem Strom auch die in der Quenchkolonne K 1000 nicht kondensierte MAS-Menge enthalten sein muß, pegelte sich MAS in der aus der K 2000 ablaufenden Lösung auf. Bei einer Frischwassermenge von ungefähr 5kg/h ergab sich eine MAS-Konzentration von ungefähr 15 Gew.-% im Ablauf aus der Absorptionskolonne K 2000, die die MAC-Löslichkeit in der Kreislauflösung (7) für die MAC-Absorption stark verbesserte.

Die Methacrylsäure wurde aus dem Ausschleusestrom (21) mit einer dreistufigen Mixer-Settler-Kaskade mit der als Edukt für die MAS-Synthese verwendeten Methacroleinlösung (2,9 kg/h) (1) extrahiert.

Um das im wäßrigen Raffinat (22) gelöste MAC zurückzugewinnen, wurde in der Stripp-Kolonne K 2100 (Durchmesser 50 mm, 1000 mm Sulzer CY-Packung) mit einem Teil der zur Oxidation verwendeten Luft (8/8b) MAC fast vollständig gestrippt. Analog dazu wurde mit der Kreislauflösung (7/7a) in Stripper K 2200 verfahren.

Die mit ungefähr 23 Gew.-% MAS beladene Methacrolein-Lösung (23) (organische Phase) ließ sich in der Mixer-Settler-Kaskade gut von der wäßrigen Phase trennen und wurde zur Auftrennung in den Flüssigkeitskreislauf (6a, 6) der Quenchkolonne K 1000 gefahren. In dieser wurde das Methacrolein aus der Extraktion verdampft, während die Methacryl- und Essigsäure in der flüssigen Phase blieben.

Die für die Abgaswäsche verwendete Kreislauflösung (7) wurde in der Stripp-Kolonne K 2200 (Durchmesser 50 mm, 1000 mm Sulzer CY-Packung) mit einem Teil der für die Oxidation benötigten Luft durch Strippung regeneriert.

Durch den MAS-Gehalt von ungefähr 15 Gew.-% mußte die Kreislauflösung (9, 9a, 9b) nur auf 15 °C abgekühlt werden, um in der Abgaskolonne K 3000 (Durchmesser 50 mm, 1000 mm Sulzer CY-Packung im Kondensationsteil (AK) und 2000 mm Sulzer CY-Packung im Absorptionsteil (MA) eine Abreicherung des Abgases (16) auf < 1000 ppm Methacrolein zu erreichen. Die mit über 4 Gew.-% MAC beladene Kreislauflösung (19) wurde in den Wärmeüberträgern W 3200 und W 3300 auf ungefähr 62°C erwärmt und in den unteren Teil der Kreisgasabsorptionskolonne K 2000 dosiert. Hier wurde der Großteil des aus dem Abgas absorbierten (Meth)acroleins in das Kreisgas (2) gestrippt.

Für eine bessere MAC-Absorption wurde das Abgas (12) im unteren Teil der Abgaskolonne K 3000 auf ungefähr 30°C abgekühlt (AK). Dabei wurden die im Abgas enthaltenen Säuren zum größten Teil kondensiert (14, 14a).

Wie sich aus dem vorstehenden ergibt, weist das erfindungsgemäße Verfahren insbesondere die folgenden Vorteile auf:
- Die Extraktion der (Meth)acrylsäure kann ohne Zusatz von Fremdstoffen durchgeführt werden;
- das Verfahren kann ohne Destillationsstufen, die energieintensiv sind, durchgeführt werden, wobei eine bis zu 100 gew.-%ige (Meth)acrylsäure-Lösung erhalten wird.

## Patentansprüche

1. Verfahren zur Herstellung von (Meth)acrylsäure, das die folgenden Stufen umfaßt:
A: Herstellung eines Reaktionsgases, das (Meth)acrylsäure, ein Alkan, Alkanol, Alken, oder Alkenal mit 3 oder 4 C-Atomen oder ein Gemisch aus zwei oder mehr davon, Wasser und Sauerstoff enthält, durch heterogen-katalysierte Gasphasenoxidation eines Alkans, Alkanols, Alkens oder Alkenals mit 3 oder 4 C-Atomen oder einem Gemisch aus zwei oder mehr davon;
B: Kondensation des in Stufe A erhaltenen Reaktionsgases, wobei eine erste wäßrige Lösung, die die Hauptmenge an (Meth)acrylsäure und eine Restmenge an Alkan, Alkanol, Alken, Alkenal mit 3 oder 4 C-Atomen oder des Gemischs aus zwei oder mehr davon aus dem Reaktionsgas enthält, und eine erste Gasphase, die die Hauptmenge an Alkan, Alkanol, Alken, Alkenal mit 3 oder 4 C-Atomen oder des Gemischs aus zwei oder mehr davon und eine Restmenge an (Meth)acrylsäure aus dem Reaktionsgas enthält, erhalten wird;
C: Absorption der in der ersten Gasphase enthaltenen (Meth)acrylsäure durch Inkontaktbringen der ersten Gasphase mit Frischwasser, wobei eine zweite wäßrige Lösung, die (Meth)acrylsäure enthält, und eine zweite Gasphase, die das Alkan, Alkanol, Alken oder Alkenal mit 3 oder 4 C-Atomen oder das Gemisch aus zwei oder mehr davon enthält, erhalten wird;
D: Extraktion der zweiten wäßrigen Lösung, die (Meth)acrylsäure enthält, durch Inkontaktbringen mit einer Lösung, die ein Alkan, Alkanol, Alken oder Alkenal mit 3 oder 4 C-Atomen oder ein Gemisch aus zwei oder mehr davon enthält, und mit der zweiten wäßrigen Lösung eine Mischungslücke bildet, wobei eine organische Phase, die (Meth)acrylsäure und den Hauptteil an Alkan, Alkanol, Alken, Alkenal mit 3 oder 4 C-Atomen oder des Gemischs aus zwei oder mehr davon enthält, und eine dritte wäßrige Lösung, die einen geringeren Teil an Alkan, Alkanol, Alken, Alkenal mit 3 oder 4 C-Atomen oder dem Gemisch aus zwei oder mehr davon enthält, erhalten wird.

2. Verfahren nach Anspruch 1, wobei die erste wäßrige Lösung vom darin enthaltenen Alkan, Alkanol, Alken, Alkenal mit 3 oder 4 C-Atomen oder dem Gemisch aus zwei oder mehr davon befreit wird, und eine wäßrige (Meth)acrylsäure-Lösung mit einem Gehalt an (Meth)acrylsäure von bis zu 100 Gew.-% und eine dritte Gasphase, die Alkan, Alkanol, Alken, Alkenal mit 3 oder 4 C-Atomen oder dem Gemisch aus zwei oder mehr davon enthält, erhalten wird.

3. Verfahren nach Anspruch 2, wobei die dritte Gasphase in die Kondensationsstufe B oder die Absorptionsstufe C eingebracht wird.

4. Verfahren nach einem der Ansprüche 1 bis 3, wobei die erste Gasphase oder die erste und die dritte Gasphase vor der Absorption gemäß Stufe C in einen Kreisgasstrom, der in die Absorptionsstufe C eingebracht wird, und einen Abgasstrom aufgeteilt wird.

5. Verfahren nach Anspruch 4, das die folgende zusätzliche Stufe umfaßt:
E: Kondensation des Abgasstroms, wobei eine vierte wäßrige Lösung, die die Hauptmenge an (Meth)acrylsäure und eine Restmenge an Alkan, Alkanol, Alken, Alkenal mit 3 oder 4 C-Atomen oder des Gemischs aus zwei oder mehr davon aus dem Abgasstrom enthält, und eine vierte Gasphase, die die Hauptmenge an Alkan, Alkanol, Alken, Alkenal mit 3 oder 4 C-Atomen oder des Gemischs aus zwei oder mehr davon und eine Restmenge an (Meth)acrylsäure aus dem Abgasstrom enthält, erhalten wird.

6. Verfahren nach Anspruch 5, das die folgende zusätzliche Stufe umfaßt:
F: Absorption des Alkans, Alkanols, Alkens oder Alkerats mit 3 oder 4 C-Atomen oder des Gemischs aus zwei oder mehr davon aus der vierten Gasphase durch Inkontaktbringen mit einer fünften wäßrigen Lösung, die (Meth)acrylsäure enthält, wobei ein Abgas, das im wesentlichen frei von (Meth)acrylsäure und Alkan, Alkanol, Alken Alkenal mit 3 oder 4 C-Atomen oder dem Gemisch aus zwei oder mehr davon ist, und eine beladene Kreislauflösung, die (Meth)acrylsäure und Alkan, Alkanol, Alken, Alkenal mit 3 oder 4 C-Atomen oder ein Gemisch aus zwei oder mehr davon enthält, erhalten wird.

7. Verfahren nach einem der Ansprüche 4 bis 6, das die folgende zusätzliche Stufe umfaßt:
G: Desorption des Alkans, Alkanols, Alkens oder Alkenals mit 3 oder 4 C-Atomen oder des Gemischs aus zwei oder mehr davon aus der beladenen Kreislauflösung durch Inkontaktbringen mit einem Gas, das das Alkan, Alkanol, Alken oder Alkenal mit 3 oder 4 C-Atomen oder das Gemisch aus zwei oder mehr davon enthält, vorzugsweise mit dem Kreisgasstrom, wobei eine sechste wäßrige Lösung, die (Meth)acrylsäure enthält, und eine fünfte Gasphase, die das Alkan, Alkanol, Alken oder Alkenal mit 3 oder 4 C-Atomen oder das Gemisch aus zwei oder mehr davon enthält, erhalten wird.

8. Verfahren nach einem der Ansprüche 1 bis 7, wobei die vierte wäßrige Lösung, die Kreislauflösung oder die sechste wäßrige Lösung, die jeweils (Meth)acrylsäure enthalten, einzeln, als Kombination von zwei oder mehr davon, oder zusammen mit der zweiten wäßrigen Lösung mindestens einen Zulauf bilden, der in die Extraktionsstufe D eingebracht wird.

9. Verfahren nach einem der Ansprüche 1 bis 8, wobei die zweite wäßrige Lösung, die (Meth)acrylsäure enthält, oder der mindestens eine Zulauf oder eine Kombination aus zwei oder mehr davon vor der Extraktion gemäß Stufe D in einen Strom zur Wasserausschleusung, der in die Extraktionsstufe D eingebracht wird, und eine Kreislauflösung für die Abgaswäsche aufgeteilt wird.

10. Verfahren nach einem der Ansprüche 1 bis 9, wobei die in der Extraktionsstufe D erhaltene organische Phase in die Kondensationsstufe B eingebracht wird.

11. Verfahren nach einem der Ansprüche 1 bis 10, wobei die Extraktion gemäß Stufe D mit einer Lösung durchgeführt wird, die ein Alkan, Alkanol, Alken, oder Alkenal mit 3 oder 4 C-Atomen oder ein Gemisch aus zwei oder mehr davon enthält, durchgeführt wird, und als Edukt für die Herstellung von (Meth)acrylsäure gemäß Stufe A eingesetzt wird und vorzugsweise eine Konzentration an Alkan, Alkanol, Alken, Alkenal mit 3 oder 4 C-Atomen oder dem Gemisch aus zwei oder mehr davon von 50 bis 100 Gew.-% besitzt.

12. Verfahren nach einem der Ansprüche 1 bis 11, wobei die dritte wäßrige Lösung, die das Alkan, Alkanol, Alken oder Alkenal mit 3 oder 4 C-Atomen oder das Gemisch aus zwei oder mehr davon enthält, mit einem Inertgas in Kontakt gebracht wird, wobei ein im wesentlichen von (Meth)acrylsäure und Alkan, Alkanol, Alken, Alkenal mit 3 oder 4 C-Atomen oder dem Gemisch aus zwei oder mehr davon freies Abwasser und eine sechste Gasphase, die Alkan, Alkanol, Alken, Alkenal mit 3 oder 4 C-Atomen oder ein Gemisch aus zwei oder mehr davon enthält, erhalten wird.

13. Verfahren nach einem der Ansprüche 9 bis 12, wobei die Kreislauflösung für die Abgaswäsche durch Inkontaktbringen mit einem Inertgas regeneriert wird, wobei eine regenerierte Kreislauflösung und eine siebte Gasphase, die Alkan, Alkanol, Alken, Alkenal mit 3 oder 4 C-Atomen oder ein Gemisch aus zwei oder mehr davon enthält, erhalten wird.

14. Verfahren nach einem der Ansprüche 9 bis 13, wobei die Kreislauflösung oder die regenerierte Kreislauflösung für die Abgaswäsche als fünfte wäßrige Lösung, die (Meth)acrylsäure enthält, wie in Anspruch 6 definiert, in die Absorptionsstufe F für das Alkan, Alkanol, Alken, Alkenal mit 3 oder 4 C-Atomen oder das Gemisch aus zwei oder mehr davon eingebracht wird.

15. Verfahren nach einem der Ansprüche 1 bis 14, wobei die erste, zweite, dritte, fünfte, sechste oder siebte Gasphase, die jeweils Alkan, Alkanol, Alken, Alkenal mit 3 oder 4 C-Atomen oder ein Gemisch aus zwei oder mehr davon enthalten, einzeln oder als Kombination von zwei oder mehr davon in die Herstellungsstufe A eingebracht werden.
